# EUROPEAN PATENT SPECIFICATION

(11) **EP 1 598 079 B1**
(45) Date of publication and mention of the grant of the patent: **22.04.2009**
(21) Application number: 04713978.7
(22) Date of filing: 24.02.2004
(51) Int. Cl.: A61K 45/00, A61K 31/275, A61K 31/352, A61P 17/00, A61P 37/08

(54) **REMEDIES FOR ALLERGIC CONTACT DERMATITIS**
MITTEL ZUR BEHANDLUNG DER ALLERGISCHEN KONTAKTDERMATITIS
TRAITEMENT DE LA DERMATITE DE CONTACT ALLERGIQUE

(30) Priority: 26.02.2003 US 449752 P
(43) Date of publication of application: 23.11.2005
(73) Proprietor: Kowa Co., Ltd., Nagoya-shi, Aichi 460-8625 (JP)
(72) Inventor: FURUE, Masutaka, 8140006 (JP); UCHI, Hiroshi, 8190162 (JP); NAKAHARA, Takeshi, Fukuoka-shi, Fukuoka 8120054 (JP)
(74) Representative: Hartz, Nikolai
(86) International application number: PCT/JP2004/002114
(87) International publication number: WO 2004/075915

(56) References cited:
- WO-A-03/077914
- WO-A2-02/102232
- JP-A- 10 512 878
- JP-A- 2002 516 325
- YANO S ET AL: "Interleukin 15 induces the signals of epidermal proliferation through ERK and PI 3-kinase in a human epidermal keratinocyte cell line, HaCaT" BIOCHEMICAL AND BIOPHYSICAL RESEARCH COMMUNICATIONS 21 FEB 2003 UNITED STATES, vol. 301, no. 4, 21 February 2003 (2003-02-21), pages 841-847, XP002363605 ISSN: 0006-291X
- FAVATA M F, ET AL: 'Identification of a novel inhibitor of mitogen-activated protein kinase kinase' JOURNAL OF BIOLOGICAL CHEMISTRY vol. 273, no. 29, 1998, pages 18623 - 18632, XP002141538

## Description

### Technical Field

This invention relates to the use of an MEK inhibitor for treating allergic contact dermatitis.

### Background Art

Epidermal cells are well known to play an important role in allergic dermatitis. More specifically, keratinocytes are activated by the stimuli of antigen and chemical substance to produce various cytokines such as granulocyte/macrophage colony-stimulating factor (GM-CSF) (Lee et al., J Immunol, 159: 5084-5088 (1997); and Steinhoff et al., Curr Opin Allergy Clin Immunol, 1: 469-476 (2001)). The thus produced cytokines activate Langerhans' cell which is an antigen presenting cell (Katz et al., Nature, 282: 324-326 (1979)), and the activated Langerhans' cell moves to the regional lymph node where it presents the antigen to naive T lymphocyte (Kripke et al., J Immunol, 145: 2833-2838 (1990); and Banchereau et al., Annu Rev Immunol, 18: 767-811 (2000)).

Increase in GM-CSF has been reported for the patients of atopic dermatitis (Pastore et al., J Clin Invest, 99: 3009-3017 (1997)), and it has been conceived that GM-CSF from keratinocyte acts on Langerhans' cell to enhance its immunostimulative function (Witmer-Pack et al., J Exp Med, 166: 1484-1498 (1987); Heufler et al., J Exp Med, 167: 700-705 (1988); and Salgado et al., J Invest Dermatol, 113: 1021-1027 (1999)) and simultaneously suppresses production of IL-12 which is a substance that induces type 1 helper T cell involved in the contact dermatitis (Toda et al., J Immunol, 164: 5113-5119 (2000)) to induce immune response by the type 2 helper T cell in the atopic dermatitis patients. Extracellular regulated kinase (ERK) which is one of the mitogen-activated protein kinase (MAPK) is also known to be involved in the production of GM-CSF (Kimata et al., Biochem Pharmacol, 60: 589-594 (2000); Hallsworth et al., Am J Respir Crit Care Med, 164: 688-697 (2001); and Dumitru et al., Cell, 103: 1071-1083 (2000)).

The signal transduction mediated by the MAPKs system takes specific pathways involving specific MAPK kinases, and exemplary signal transduction pathway includes the route from MEK1/2 via ERK1/2, the route from MKK7 and MKK4/SEK1 via JNK, and the route from MKK3/6 via p38 (Yamanaka et al., Experimental medicine, 20:206-210 (2002)). With regard to the interaction of ERK and p38, MEK/ERK is believed to act competitively with p38 in view of the facts that DNA synthesis in rat lung myofibroblast by cell propagation stimulus and activation of ERK1/2 are enhanced by SB203580 which is the selective inhibitor for p38 (Rice et al., Am J Respir Cell Mol. Biol, 27: 759-765 (2002)), and that, in the formation of osteoclast, SB203580 and PD169316 which are specific inhibitors for the p38 exhibit suppressive action while U0126 and PD98059 which are specific inhibitors for the MEK exhibit promotive action, and that phosphorylation of the ERK is enhanced by the p38 inhibitor, while phosphorylation of the p38 is enhanced by the MEK inhibitor (Hotokezaka et al., J Biol Chem, 277: 47366-47372 (2002)).

These findings indicate that the MEK/ERK-mediated pathway is involved in the onset mechanism of the atopic dermatitis, while GM-CSF, whose production is mediated by MEK/ERK, suppresses induction of Th1 which is involved in the allergic contact dermatitis. As described above, the onset mechanism of the atopic dermatitis is clearly different from that of the contact dermatitis, and accordingly, development of the therapeutic drugs targeting these two diseases had to be conducted from a different point of view.

### Disclosure of the Invention

While various drugs have been developed for atopic dermatitis, only few effective agents are available for contact dermatitis. Accordingly, an object of the present invention is to provide a therapeutic agent for treating allergic contact dermatitis.

The inventors of the present invention made an extensive investigation in search of drugs effective in treating the allergic contact dermatitis, and quite unexpectedly found that an MEK inhibitor exhibits excellent therapeutic effects in mouse model of picryl chloride-induced dermatitis which is widely used as a model for allergic contact dermatitis, while MEK/ERK inhibitor has been conceived to be effective for atopic dermatitis but not for the contact dermatitis since action of the MEK/ERK is competitive to p38. The present invention has been completed on the basis of such a finding.

This invention provides use of a substance having MEK inhibitory action for production of a therapeutic agent for treating allergic contact dermatitis.

### Brief Description of the Drawings

FIG. 1 is a view showing the therapeutic effects of U0126 for allergic contact dermatitis. The results are represented as average ± standard error. (*: p < 0.05, N = 8)
FIG. 2 is a view showing the therapeutic effects of PD98059 for allergic contact dermatitis. The results are represented as average ± standard error. (*: p < 0.05, **: p < 0.01)

### Best Mode for Carrying Out the Invention

The effective component of the drug for treating allergic contact dermatitis of the present invention is not particularly limited as long as it is a substance having an inhibitory action for MEK, and exemplary such components include known MEK inhibitors such as bis[amino[(2-aminophenyl)thio]methylene]butanedinitrile (U0126, J. Biol. Chem., Vol. 273, No. 29, pages 18623-18632 (1998)) and 2-(2-amino-3-methoxyphenyl)-4-oxo-4H-[1]benzopyran (PD98059, USP 5, 525, 625) .

These MEK inhibitors may be used in the form of a salt, and the salt is not particularly limited as long as it is a pharmaceutically acceptable salt. Exemplary salts include acid adduct salts of a mineral acid such as hydrochloride, hydrobromide, hydroiodide, sulphate, and phosphate; and acid adduct salts of an organic acid such as benzoate, methanesulphonate, ethanesulphonate, benzenesulphonate, p-toluenesulphonate, succinate, maleate, fumarate, tartarate, citrate, and acetate. Among these, the preferred are hydrochloride, sulphate, maleate, and fumarate.

These MEK inhibitors may also exist in the form of a solvate as typically represented by hydrate. Such solvate is also within the scope of the invention.

These MEK inhibitors exhibit excellent therapeutic effects for allergic contact dermatitis as will be demonstrated in the Examples, and they are quite useful as a drug for treating contact dermatitis such as skin inflammation caused by immediate response as well as delayed response which are induced by the contact with a chemical substance, nickel, rubber, or other minerals, animals, plants, fungi, and the like.

The drug for treating allergic contact dermatitis of the present invention contains a MEK inhibitor as its effective component, administration form is not particularly limited and any adequate form may be selected depending on the object of the treatment. Exemplary dosage forms include oral preparation, injection, suppository, and also, percutaneous preparation, inhalant, eye drops, nasal drops, ear drops, and other external preparations, and the composition well suited for such administration form may be produced by any of the common methods known in the art by incorporating a pharmaceutically acceptable carrier.

When an oral solid preparation is prepared, the MEK inhibitor may be admixed with an excipient, and optionally, with a binder, a disintegrant, a lubricant, a colorant, a taste corrective, a flavor corrective, or the like, and the mixture may be produced into tablets, coated tablets, granules, powder, capsules, or the like by the method commonly used in the art. The additives used may be those commonly used in the art, and exemplary excipients include lactose, sugar, sodium chloride, glucose, starch, calcium carbonate, kaolin, microcrystalline cellulose, and silicic acid, and exemplary binders include water, ethanol, propanol, simple syrup, glucose solution, starch solution, gelatin solution, carboxymethylcellulose, hydroxypropyl cellulose, hydroxypropyl starch, methylcellulose, ethylcellulose, shellac, sodium phosphate, and polyvinylpyrrolidone. Exemplary disintegrants include dry starch, sodium arginate, agar powder, sodium hydrogenccarbonate, calcium carbonate, sodium laurylsulfate, monoglyceride stearate, and lactose, and exemplary lubricants include purified talk, stearate, borax, and polyethyleneglycol. Exemplary colorants include β-carotene, yellow ferric oxide, and caramel, and exemplary taste correctives include white sugar, orange peel, citric acid, and tartaric acid. Exemplary flavor corrective include diatomaceous earth, and kaolin.

When an oral liquid preparation is prepared, the MEK inhibitor may be admixed with a taste corrective, a buffer, a stabilizer, a flavor corrective, a preservative, or the like, and the mixture may be produced into a liquid preparation, a syrup, a elixir, or the like by the method commonly used in the art. In this case, the taste and flavor correctives may be selected from those described above, and the buffer used may be sodium citrate or the like. Exemplary stabilizers include gum traganth, gum arabic, and gelatin, and exemplary preservatives include methyl paraoxybenzoate, ethyl paraoxybenzoate, and propyl paraoxybenzoate.

When an injection is prepared, the MEK inhibitor may be admixed with a pH adjusting agent, a buffer, a stabilizer, an isotonic agent, a topical anesthetic, or the like, and the mixture may be produced into subcutaneous, intramuscular, or intravenous injection by the method commonly used in the art. In this case, the pH adjusting agent and the buffer may be sodium citrate, sodium acetate, sodium phosphate, or the like, and exemplary stabilizers include sodium pyrosulfite, EDTA, thioglycolic acid, and thiolactic acid. Exemplary isotonic agents include sodium chloride and glucose, and exemplary topical anesthetics include procaine hydrochloride, and lidocaine hydrochloride.

When a suppository is prepared, the MEK inhibitor may be admixed with a pharmaceutical carrier known in the art such as polyethyleneglycol, lanoline, cacao butter, or a fatty acid triglyceride, and if desired, further with a surfactant such as Tween (TM), and produced into the desired preparation by the method commonly used in the art.

When an external preparation such as ointment, cream, gel, lotion, solution, water-based poultice, or non-water-based poultice is produced, the MEK inhibitor may be admixed with a base, a water-soluble polymer, a crosslinking agent, a tackifier, a solvent, a stabilizer, a surfactant, a pH adjusting agent, a preservative, or the like commonly used with the MEK inhibitor, and the mixture may be stirred and produced into the desired preparation by the method commonly used in the art. Exemplary bases include purified lanoline, liquid paraffin, white petrolatum, white Beeswax, octyldodecyl alcohol, and paraffin, and exemplary water-soluble polymers include carboxyvinyl polymer, pullulan, sodium carboxy methyl cellose, sodium arginate, and xanthan gum. Exemplary crosslinking agents include dihydroxyaluminum amino acetate, and dried aluminum hydroxide gel, and exemplary tackifiers include partially neutralized polyacrylic, poly(sodium acrylate), poly(2-ethylhexyl acrylate), and styreneisoprene-styrene block copolymer. Exemplary solvents include glycerin, 1,3-butylene glycol, water, oleic acid, castor oil, and benzylalcohol, and exemplary surfactants include sodium lauryl sulfate, glyceryl monostearate, sucrose fatty ester, and polyoxyethylene fatty ester. Exemplary pH adjusting agents include sodium hydroxide, and citric acid, and exemplary preservatives include methyl paraoxybenzoate, ethyl paraoxybenzoate, and propyl paraoxybenzoate.

When eye drops are prepared, the MEK inhibitor may be admixed with a pH adjusting agent, a stabilizer, an isotonic agent, a preservative, or the like, and produced into the desired preparation by the method commonly used in the art. The additives used may be those commonly used in the art, and exemplary pH adjusting agent is sodium phosphate, and exemplary stabilizers include sodium pyrosulfite and EDTA. An exemplary isotonic agent is sodium chloride, and an exemplary preservative is chlorobutanol.

When nasal drops are prepared, the MEK inhibitor may be admixed with a pH adjusting agent, a stabilizer, an isotonic agent, a preservative, or the like, and produced into the desired preparation by the method commonly used in the art. The additives used may be those commonly used in the art, and an exemplary pH adjusting agents is sodium phosphate, and exemplary stabilizers include sodium pyrosulfite and EDTA. An exemplary isotonic agent is sodium chloride, and an exemplary preservative is benzalkonium chloride.

When ear drops are prepared, the MEK inhibitor may be admixed with a pH adjusting agent, a buffer, a stabilizer, an isotonic agent, a preservative, or the like, and produced into the desired preparation by the method commonly used in the art. The additives used may be those commonly used in the art, and exemplary pH adjusting agents and buffers include sodium phosphate, and exemplary stabilizers include sodium pyrosulfite and EDTA. An exemplary isotonic agent is sodium chloride, and an exemplary isotonic agent is benzalkonium chloride.

The drug for treating allergic contact dermatitis of the present invention may be administered at a dose which may vary according to the age, body weight, symptoms, administration form, frequency of the administration, and the like. However, the drug is generally administered orally or parenterally at 1 to 1000 mg as a MEK inhibitor per day per adult in one to several dosage(s).

### Examples

Next, the present invention is described in further detail by referring to the Examples.

### Example 1

### A. Test method

### (1) Sensitization and induction of the dermatitis

Female BALB/C mice purchased from Charles River Japan, Inc. were used. The mice were sensitized by spreading 0.1 mL of 7% picryl chloride (PC) solution in acetone on shaved abdomen, and 7 days after the sensitization, dermatitis was induced by spreading 0.01 mL of 1% PC solution in acetone on the front and the back of the left auricle (i.e. 0.02 mL in total).

### (2) Measurement of the dermatitis

Thickness of the auricle was measured before the dermatitis induction and 24 hours after the dermatitis induction, and the difference in the thickness was measured.

### (3) Preparation of the test substance and its administration

The test substance used was U0126 which is known as a specific MEK inhibitor. U0126 was used by dissolving in dimethylsulfoxide. The test substance was applied on both front and back of the left auricle at an amount of 0.02 mL (0.04 mL in total) 1 hour before the dermatitis induction.

### (4) Data processing

The results are represented as average ± standard error. Student's t test was used for the determination of the significant difference, and significance level of less than 5% was considered significant.

### B. Test results

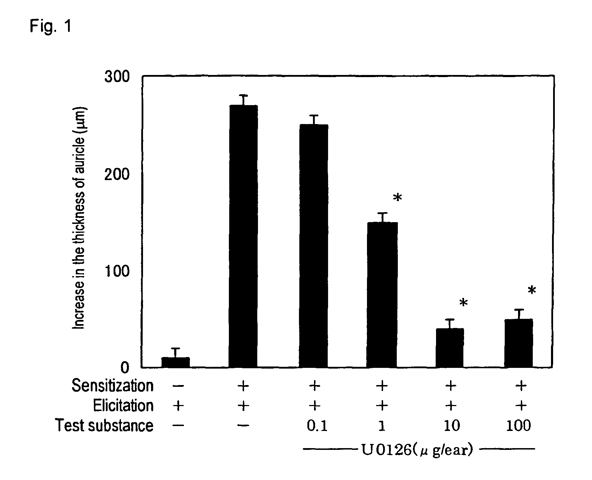

As shown in FIG. 1, U0126 (a specific inhibitor for MEK1/2 which activates extracellular regulated kinase (ERK1/2) which is a member of Mitogen-activated protein kinase (MAPKs)) exhibited suppressive action for allergic contact dermatitis in a dose dependent manner in the range of 0.1 to 10 µg/ear, and the action at 100 µg/ear was substantially the same level as the action of 10 µg/ear.

### Example 2

### A. Test method

### (1) Sensitization and induction of the dermatitis

The procedure of Example 1 was repeated.

### (2) Measurement of the dermatitis

The procedure of Example 1 was repeated.

### (3) Preparation of the test substance and its administration

The procedure of Example 1 was repeated by replacing U0126 with PD98059.

### B. Test results

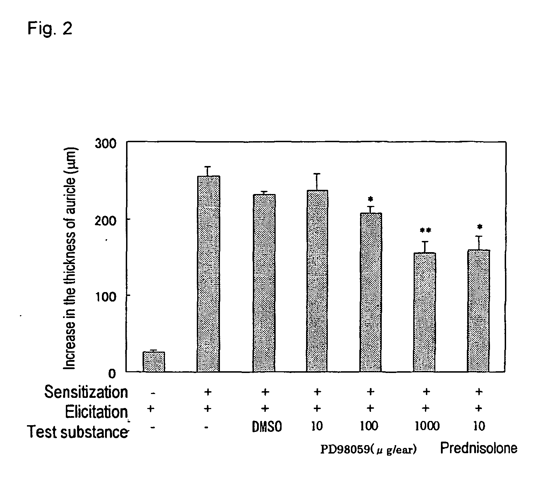

As shown in FIG. 2, PD98059 exhibited suppressive action for allergic contact dermatitis.

## Claims

1. Use of a substance having MEK inhibitory action for production of a therapeutic agent for treating allergic contact dermatitis.

2. Use according to claim 1, wherein the substance having MEK inhibitory action is bis[amino[(2-aminophenyl)thio]methylene]butanedinitrile or 2-(2-amino-3-methoxyphenyl)-4-oxo-4H-[1]benzopyran.

3. Use according to claim 1 or 2 wherein the agent is administered by external administration.

4. Use according to claim 1 or 2 wherein the agent is administered by percutaneous administration, inhalation, eye instillation, nasal instillation, or ear instillation.

## Patentansprüche

1. Verwendung einer Substanz mit MEK inhibierender Wirkung zur Herstellung eines therapeutischen Mittels zur Behandlung von allergischer Kontaktdermatitis.

2. Die Verwendung gemäß Anspruch 1, wobei die Substanz mit MEK inhibierender Wirkung Bis[amino[(2-aminophenyl)thio] methylen] butandinitril oder 2-(2-amino-3-methoxyphenyl)-4-oxo-4H-[1] benzopyran ist.

3. Die Verwendung gemäß Anspruch 1 oder 2, wobei das Mittel äußerlich verabreicht wird.

4. Die Verwendung gemäß Anspruch 1 oder 2, wobei das Mittel perkutan, durch Inhalation, durch Einträufelung in die Augen, Einträufelung in die Nase oder Einträufelung in die Ohren verabreicht wird.

## Revendications

1. Utilisation d'une substance ayant une action d'inhibition de la MEK pour la production d'un agent thérapeutique pour le traitement de la dermatite de contact allergique.

2. Utilisation selon la revendication 1, dans laquelle la substance ayant une action d'inhibition de la MEK est le bis[amino[(2-aminophényl)thio] méthylène]butanedinitrile ou le 2-(2-amino-3-méthoxyphényl)-4-oxo-4H-[1]benzopyrane.

3. Utilisation selon la revendication 1 ou 2, dans laquelle l'agent est administré par administration externe.

4. Utilisation selon la revendication 1 ou 2, dans laquelle l'agent est administré par administration percutanée, inhalation, instillation dans l'oeil, instillation nasale ou instillation dans l'oreille.
